Europäisches Patentamt

**European Patent Office**

**Office européen des brevets**

(11) Publication number: **0 242 979**
**A2**

# (12) EUROPEAN PATENT APPLICATION

(21) Application number: **87302294.1**

(22) Date of filing: **18.03.87**

(51) Int. Cl.4: **A61M 16/10**

(30) Priority: **19.04.86 GB 8609612**

(43) Date of publication of application:
**28.10.87 Bulletin 87/44**

(84) Designated Contracting States:
**BE CH DE ES FR IT LI NL SE**

(71) Applicant: **The BOC Group plc**
**Chertsey Road**
**Windlesham Surrey GU20 6HJ(GB)**

(72) Inventor: **Rollins, Neil**
**12 South Edge Shann Park**
**Keighley Yorkshire(GB)**

(74) Representative: **Gough, Peter**
**c/o THE BOC GROUP PLC Patent Department**
**Chertsey Road**
**Windlesham Surrey GU20 6HJ(GB)**

(54) **Improvements in devices for filling anaesthetic vaporisers.**

(57) A device I for filling an anaesthetic vaporiser with a volatile liquid anaesthetic includes two tubes 7, I0 tube 7 being used to convey liquid anaesthetic from a container to a vaporiser and tube I0 permitting the flow of air and/or anaesthetic gas from the vaporiser to the container.

A one-way valve assembly is provided for permitting the passage through the air tube I0 of air and/or anaesthetic gas only from the vaporiser towards the container thereby preventing or inhibiting the possibility of the air tube I0 being filled with liquid anaesthetic.

FIG. 1.

EP 0 242 979 A2

## IMPROVEMENTS IN DEVICES FOR FILLING ANAESTHETIC VAPORISERS

The present invention relates to devices for filling anaesthetic vaporisers with volatile liquid anaesthetic.

Throughout this specification the term "anaesthetic" is intended to embrace anaesthetic and analgesic agents.

As explained in U.K. Patent No. II93241 anaesthetic vaporisers are used with anaesthesia machines for mixing the vapour of a volatile liquid anaesthetic with a carrier gas such as air, oxygen, nitrous oxide or a combination thereof.

Usually an anaesthetic vaporiser is dedicated for use with only one type of anaesthetic agent and U.K. Patent No. II93241 describes a filling system which substantially eleminates the possibility of an anaesthetic vaporiser being filled with the wrong anaesthetic agent.

This know system uses separate tubes which respectively convey liquid anaesthetic from a container, usually a bottle, to the anaesthetic vaporiser (known as the liquid tube) and air and/or anaesthetic gas from the vaporiser to the container (known as the air tube) under the action of gravitational forces.

Although this known system is widely used by hospital technicans with a great deal of success, in certain circumstances the system does not function efficiently because the air tube becomes filled or partly filled with liquid anaesthetic to such an extent that air and/or anaesthetic gas will not flow through the air tube.

It is an aim of the present invention to provide a device for filling an anaesthetic vaporiser with a volatile liquid anaesthetic from a container which minimises the possibility of the air tube becoming filled or partly filled with liquid anaesthetic.

According to the present invention, a device for filling an anaesthetic vaporiser with a volatile liquid anaesthetic from a container comprises a first tube formed with an inlet at or adjacent one end and communicating at its opposite end with an outlet port formed in an outlet member for the passage therethrough of liquid anaesthetic, a second tube formed with an outlet at or adjacent one end and communicating at its opposite end with an inlet port formed in said outlet member, a one-way valve for permitting the passage through the second tube of air and/or anaesthetic gas from the inlet port towards said outlet, the outlet member being adapted to fit in a filler socket forming part of an anaesthetic vaporiser to be filled, and a cap connection adapted to be secured to the anaesthetic container and arranged such that when so secured the inlet of the first tube and the outlet of the second tube are in communication with the interior of the container.

Preferably, the second tube is arranged within the first tube for most of its length and terminates adjacent its outlet end in a one-way ball valve arrangement.

An embodiment of the invention will now be described by way of example, reference being made to the Figures of the accompanying diagrammatic drawings in which :-

Figure I is a side elevation, partly in cross-section, of a device for filling an anaesthetic vaporiser with a volatile liquid anaesthetic;

Figure 2 is a sectional view of the device of Figure I having an outlet member fitted within a filler socket forming part of an anaesthetic vaporiser;

Figure 3 is a sectional view of a cap connection forming part of the device of Figure I;

Figure 4 is a cross-section of a one-way ball valve forming part of the device of Figure I;

Figure 5 is a cross-section illustrating the cap connection of Figure 3 secured to a container; and

Figure 6 is a perspected view illustrating the device of Figure I being used to fill an anaesthetic vaporiser from a bottle of liquid anaesthetic.

As shown, a device I for filling an anaesthetic vaporiser 5 with a volatile liquid anaesthetic includes an outer (liquid) tube 7 and an inner (air) tube I0. At the right-hand (as shown in Figure I) end of the device I the tubes 7, I0 are secured by means of a ferrule 6 to an outlet member 2 formed with an outlet port II for liquid anaesthetic and inlet port I2 for air and/or anaesthetic gas. The liquid tube 7 communicates with outlet II and the air tube I0 communicates with the inlet port I2. The outlet member 2 is formed with keying means, for example, a slot positioned and/or dimmensioned to mate with a locating tongue or peg on a filler block 3 forming part of the anaesthetic vaporiser 5 so that only an outlet member of the correct shape can enter into an opening 4 of the filler block 3.

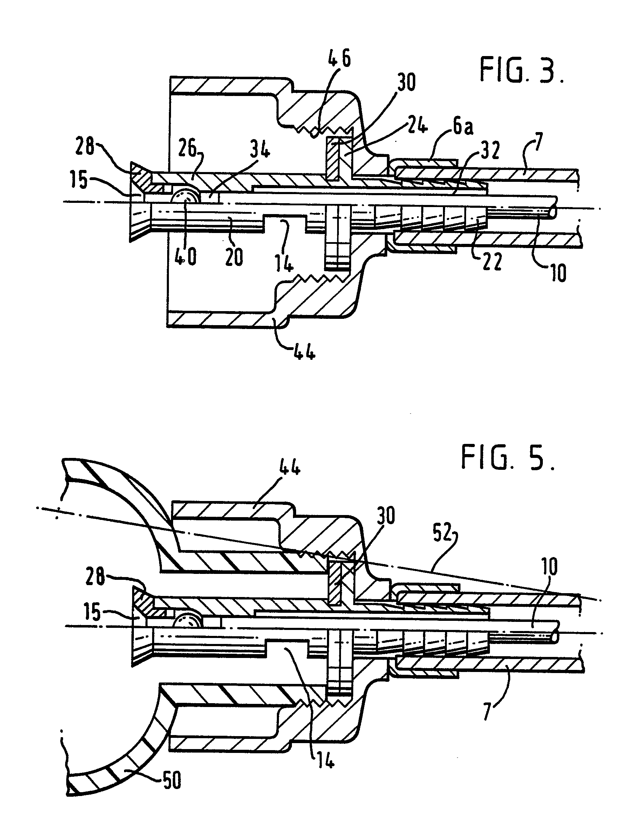

Referring in particular to Figures 3 and 4, the tubes 7, I0 at the left-hand (as shown in Figure I) end of the device I terminate at a tubular end piece assembly 20. The assembly 20 includes a main body having a tail piece 22, a laterally extending flange 24 and a forward piece 26. Secured to the free end of the forward piece 26 is a valve end 28 formed with an outlet port I5. A sealing washer 30 is positioned against the flange 24. The liquid tube 7 is secured to the tail piece 22 by a ferrule 6a.

The interior of the tubular end piece assembly 20 has a first cylindrical passage 32, a second smaller diameter cylindrical passage 34, a counter bore 36 , the counter bore 36 and the passage 34 defining a sealing surface 38 forming part of a one-way valve arrangement. The valve arrangement includes a light ball 40 movable between the sealing surface 38 and the valve end 28 in the counter bore 36. The valve end 28 is formed with protrusions 42 which leave a passage between the ball and the valve end 28 if the ball is displaced to its extreme left-hand position as shown in Figure 3. The air tube 10 terminates in the passage 34 and defines with the interior surface of the passage 32 an annular flow path for liquid anaesthetic which communicates with an inlet 14 formed in the forward piece 26.

A cap connection 44 is secured to the end piece assembly 20 having a portion trapped between the flange 24 and the ferrule 6 a. The cap connection 44 has threads 46 enabling it to be secured to a bottle 50 (See Figure 5) containing volatile liquid anaesthetic in a manner known per se.

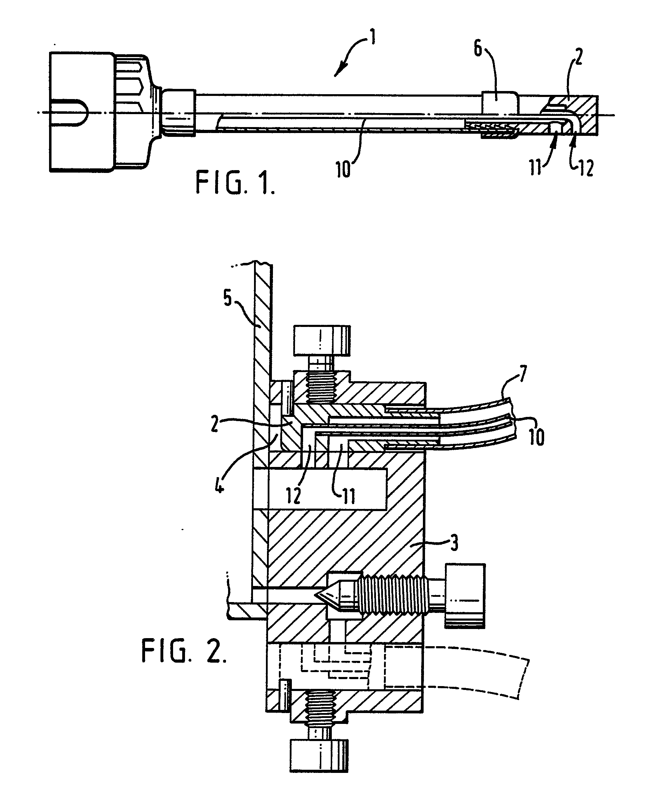

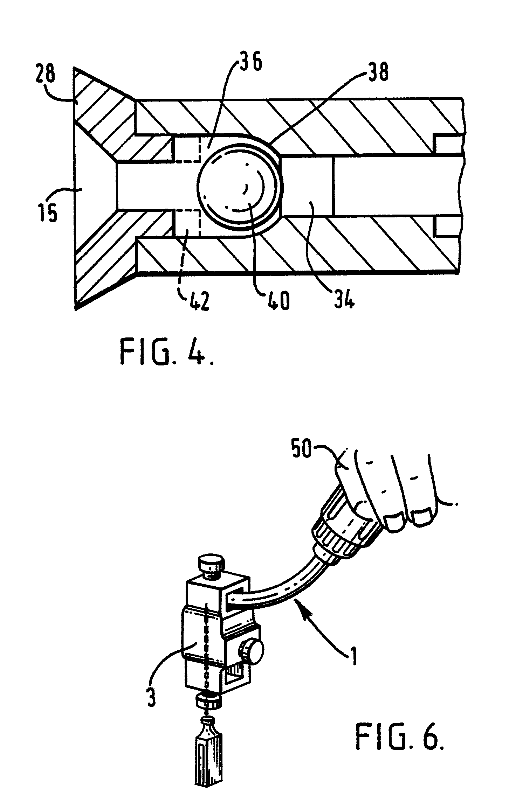

In use, the cap of the bottle containing the liquid anaesthetic is removed and the cap connection 44 is screwed to the bottle opening in an air tight manner such that the end of the bottle engages the sealing washer 30 (see Figure 5). The outlet member 2 is then inserted into the opening 4 in the filler block 3 as shown in Figure 2 and the bottle is raised above the level of the opening 4 as shown in Figure 6. As the bottle is raised the liquid level 52 (see Figure 5) progressively tilts, and liquid flows towards the inlet 14 and the outlet port 15. As liquid attempts to flow into outlet port 15 it displaces the ball 40 which engages sealing surface 38. Liquid now preferentially flows through the inlet 14. Liquid will continue to flow preferentially through the inlet 14 and this sets up a flow regime in motion whereby liquid flows down the liquid tube 7 towards the vaporiser and enters the vaporiser via outlet port 11. At the same time air and/or anaesthetic gas flows from the vaporiser through inlet port 12 along air tube 10 and displaces the lightweight ball 40 to the left as shown in Figure 3 to exit from the outlet port 15 into the bottle. Liquid is inhibited from entering the air tube 10 by a combination of the upward flow of air and the obstruction of the ball 40 and its ability to move in response to the forces exerted upon it. Once this flow has been established filling continues until either the air or liquid flow is stopped by other means.

The device 1 can be used for draining a vaporiser and as with other known devices the air and liquid are required to flow in the reverse direction to that used for filling. When the device 1 is used for draining a vaporiser, the bottle is lower than the filler block 3 and the ball 40 is displaced by gravity so that it rests against the protrusions 42. Hence air can pass around the ball 40 thus permitting reverse flow through the inner (air) tube 10.

Modifications can be made to the embodiment described above in that although a one way ball valve is described different types of one-way valve could be incorporated, for example a flap or disc type valve.

A particular advantage of the embodiment described above is that it is very simple to operate and removes the necessity for providing complicated valve systems which require accurately machined parts and hence high manufacturing costs.

## Claims

1. A device 1 for filling an anaesthetic vaporiser 5 with a volatile liquid anaesthetic from a container 50 comprising a first tube 7 formed with an inlet 14 at or adjacent one end and communicating at its opposite end with an outlet port 11 formed in an outlet member 2 for the passage therethrough of liquid anaesthetic, a second tube 10 formed with an outlet 15 at or adjacent one end and communicating at its opposite end with an inlet port 12 formed in said outlet member 2, the outlet member 2 being adapted to fit in a filler opening 4 forming part of the anaesthetic vaporiser 5 to be filled and a cap connection 44 adapted to be secured to the container 50 and arranged such that when so secured the inlet 14 of the first tube 7 and the outlet 15 of the second tube 10 are in communication with the interior of the container 50, characterised by a one-way valve 38, 40, 28 for permitting the passage through the second tube 10 of air and/or anaesthetic gas from the inlet port 12 towards said outlet 15.

2. A device as claimed in claim 1, in which the second tube 10 is arranged within the first tube 7 for most of its length, characterised in that the second tube 10 terminates adjacent its outlet 15 in a one-way ball valve arrangement 38, 40, 28.

3. A device as claimed in claim 2, characterised in that the first and second tubes 7, 10 terminate at their said one ends in a tubular end piece assembly 20 which includes a valve end 28 formed with an outlet port 15 for the passage therethrough of air and/or anaethetic gas, the valve end 28 being secured to a main body which incorporates the one-way ball valve arrangement 38, 40, 28 and an inlet 14 formed in the main body communicating with the first tube 7 for the passage therethrough of liquid anaesthetic.

FIG. 1.

FIG. 2.

FIG. 3.

FIG. 5.

FIG. 4.

FIG. 6.